Europäisches Patentamt

European Patent Office

Office européen des brevets

(11) Publication number: **0 107 886**
**B1**

## (12) EUROPEAN PATENT SPECIFICATION

(45) Date of publication of patent specification: **04.02.87**

(21) Application number: **83201531.7**

(22) Date of filing: **25.10.83**

(51) Int. Cl.⁴: **C 07 C 17/02,** C 07 C 19/045, C 01 D 7/16

(54) **Process for the combined preparation of chlorinated hydrocarbons and a carbonate of an alkali metal.**

(30) Priority: **29.10.82 NL 8204186**

(43) Date of publication of application:
**09.05.84 Bulletin 84/19**

(45) Publication of the grant of the patent:
**04.02.87 Bulletin 87/06**

(84) Designated Contracting States:
**BE DE FR GB IT NL SE**

(56) References cited:
**US-A-4 010 243**
**US-A-4 256 719**

(73) Proprietor: **AKZO N.V.**
**Velperweg 76**
**NL-6824 BM Arnhem (NL)**

(72) Inventor: **Albers, Jan**
**Huygensstraat 16**
**NL-7471 XT Goor (O) (NL)**

(74) Representative: **Sieders, René et al**
**P.O. Box 314**
**NL-6800 AH Arnhem (NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

Process for the combined preparation of chlorinated hydrocarbons and a carbonate of an alkali metal.

The invention relates to both the preparation of chlorinated hydrocarbons and the preparation of a carbonate of an alkali metal, the two preparations being intercoupled as it were, use being made in either case of one and the same auxiliary material. The term carbonate as used herein refers to both carbonate and bicarbonate.

A process for such a combined preparation is disclosed in US 4 256 719. This known process comprises the process steps mentioned in the introductory part of claim 1, the auxiliary material generally being an aliphatic amine having a boiling point below about 100°C. Such a combined preparation is also reported in Research Disclosure of September 1980, p. 365.

It has been found that in actual practice the use of the amines named in said disclosures, particularly the trimethylamine (TMA) recommended therein, is subject to rather undesirable limitations. These limitations are mainly bound up with the stability of these amines under the conditions of the reaction.

The object of combining the chlorination process (step a) with the soda process (step b) is to make it possible for the amine auxiliary material to keep serving its purpose in the two processes without any appreciable loss. Loss of amines as a result of decomposition, however, cannot altogether be avoided, so that also when use is made of TMA the oxidative regeneration of the chlorination liquid must be carried out at a considerably lower temperature (about 50°C) than the chlorination (about 100°C). Of course, also for the stripping of TMA the temperature must not be chosen too high. Nevertheless, even at relatively low thermal levels loss of TMA as a result of decomposition cannot altogether be prevented.

Apart from this inevitable loss the maintenance of various temperature levels in the chlorination cycle is necessarily attended with the energy wasting procedure of repeated cooling and heating.

It has now been found that for the purpose envisaged use can be made of a considerably stabler type of amines.

The invention consists in that in the afore-mentioned known process use is made of an amine having exclusively hydrogen-free carbon atoms in the α-position and a $k_a$ lower than 0,01 min⁻¹.

The symbol $k_a$ stands for the first order reaction rate constant in alkalinity under standard conditions and is a measure of the rate of amine decomposition.

Amines of this type appear to be sufficiently stable to be safely exposed to higher temperature than permitted with the previously known amines without being subject to decomposition.

This has the great advantage that the regeneration of the chlorinating liquid also may be carried out at elevated temperature, including the stripping of the amine recovered. As a result, the entire chlorination cycle can, if desired, by kept at the same temperature level.

Examples of suitable α-hydrogen-free amines include 2-amino-2-methyl propane (t-butylamine), di-t-butylamine, 2-amino-2-methyl propanol 1, and 2,2,6,6-tetramethyl piperidine. Preference is given to t-butylamine (TBA). In view of their considerably better thermal stability, however, there is no need for the α-H-free amines to have a boiling point below 100°C, provided that there is sufficient evaporation in the stripping section.

The favourable effect of α-H-free carbon atoms on the stability of the amines will be apparent, int.al., from the following table, in which $k_a$ is a measure of the rate of amine decomposition under standard conditions.*

| Amine | $k_a$(min⁻¹) | Temperature °C | Number of α-H's |
|---|---|---|---|
| trimethylamine | 0,135 | 80 | 9 |
| diethylamine | 0,29 | 83 | 4 |
| diisopropylamine | 0,042 | 80 | 2 |
| t-butylamine | 0,0007 | 90 | 0 |
| 2 amino 2 methyl propanol 1 | 0,0035 | 88 | 0 |
| 2,2,6,6-tetramethyl piperidine | 0,0013 | 84 | 0 |

*Test solution:

| | |
|---|---|
| 25 ml adiponitrile | 0,65 moles/kg $CuCl_2 \cdot 2$ aq. |
| 2,2 moles/kg amine HCl | 0,275 moles/kg CuCl |
| 0,5 moles/kg amine | 0,225 moles/kg $I_2$ |

# 0 107 886

Heat the solution to 80°—90°C, add amine, maintain at temperature and determine change in alkalinity (by amine decomposition) with time by potentiometric analysis and calculate from it the first order reaction rate constant $k_a$.

The chlorinating liquid is a substantially non-aqueous system containing a suitable organic solvent. Various solvents may be used, as is also mentioned in US 4 256 719. With the use of the present α-H-free amines the choice of the solvent is partly influenced by the higher temperature that can be employed. As examples of suitable solvents may be mentioned N-methyl pyrrolidone, tetramethylene sulphone, 3-methyl tetramethylene sulphone, adiponitrile and pyrrolidone-2.

The use of the α-H-free amine TBA leads to good results, especially when the solvent employed is N-methyl pyrrolidone.

The process of the invention will be further described in the following example. The examples gives a stepwise illustration of the preparation by the process of the invention of EDC from ethylene and of soda from sodium chloride.

For the combined preparation envisaged the various process steps are, evidently, intercoupled in actual practice by circulation of process flows.

Example

The experiments for regeneration (step c) and chlorination (step a) were successively carried out in the same reactor. The preparation of carbonate (step b) was conducted in a separate experimental set-up.

1. Regeneration/chlorination

—Preparation of chlorinating liquid and oxidation

— In a 2-l glass reactor fitted with a stirrer a mixture was prepared from 99 g of cuprous chloride, 219 g of t-butylamine hydrochloride (TBA.HCl), 63,4 g of iodine, 20 g of water and 598,5 g of 1 methyl 2-pyrrolidone (NMP). The mixture was heated to 100°C, after which through an inlet tube to below the level of the liquid air was introduced at a rate of 65 standard litres per hour (2,4 moles/h), a pressure of 600 kPa being maintained in the reactor. The speed of the stirrer had been so chosen that the reaction speed decreased after about 115 minutes, at which moment 700 mmoles of TB-A had formed and the air feed was stopped.

—TBA stripping

— The TBA formed was partly stripped in vacuo (4—7 kPa) with a nitrogen stream at a rate of about 150 standard litres per hour. After about 60 minutes half of the TBA formed was stripped and for the greater part condensed at −30°C. The off-gas from the condenser was washed free of TBA with dilute HCl in an absorber. The TBA (about 350 mmoles) left in the contents of the reactor was subsquently neutralized with 37%-hydrochloric acid.

—Ethylene chlorination

— In the same reactor chlorination was conducted at 100°C and 300 kPa by introducing ethylene through an inlet tube extending to below the level of the liquid. After about 60 minutes the reaction was stopped, at which moment about 350 mmoles of 1,2-dichloro-ethane (EDC) had formed.

—EDC stripping

— Next, the EDC formed was stripped with nitrogen (about 150 standard liters per hour) in vacuo at 100°C and removed from the stripping gas by condensation at −30°C.

—Restoring of reactor contents

— Finally, the contents of the reactor were restored to their original specification by adding about 350 mmoles of TBA.HCl and some NMP which had escaped during stripping. The afore-described cycle was re-started with oxidation and was carried out ten times in all to lead to practically identical results.

2. Preparation of carbonate

A 250-ml flask was charged with an aqueous solution saturated at 20°C and made up of 1,27 g of $NaHCO_3$, 29,7 g of NaCl and 31,8 g of TBA.HCl in 120 g of water. $CO_2$ was introduced at a rate of 87 mmoles/h through an inlet tube extending to below the level of the liquid. A gas mixture of nitrogen (1170 mmoles/h) and TBA (61 mmoles/h) was introduced through a second inlet tube.

In the off-gas the amount of TBA which had not been taken up was measured by absorption in an acid solution which was kept at a constant pH by titration with acid.

After 93,5 minutes the experiment was stopped, at which moment 84 mmoles of TBA had been taken up by the solution and a crystal sediment had formed. The crystal mass was filtered off and dried to the air to 7,7 g containing 81,9% by weight of $NaHCO_3$.

The crystals were washed once with water to free them of impurities introduced by the adhering mother liquor and dried at 100°C to yield 4,4 g of crystals containing 95% by weight of sodium carbonate and bicarbonate.

To the remaining mother liquor were added 84 mmoles of NaCl and 84 mmoles of water. The salt crystals slowly dissolved with stirring at room temperature and a floating crystal was formed.

After 2 hours practically all NaCl had dissolved. The crystals formed were filtered off and dried to the air to yield 11,2 g containing 78,5 % by weight of TBA.HCl. Again a single washing with water and drying at 100°C yielded 7,5 g of crystals containing 96% TBA.HCl. The residual mother liquor had the same composition as the solution with which the experiment was started.

**Claims**

1. A process for the combined preparation of chlorinated hydrocarbons and a carbonate of an alkali metal comprising the steps of

a. chlorinating an olefinic hydrocarbon with a substantially non-aqueous chlorinating liquid containing iodine and cupric and/or ferric chloride;

b. converting an alkali metal chloride by carbonation into carbonate with the aid of an alipathic amine as alkaline material, with formation of the amine hydrochloride as byproduct;

c. regenerating the chlorinating liquid used in step a. with the amine hydrochloride formed in step b. as chlorine source in the presence of oxygen or an oxygen-containing gas, and returning the amine recovered to step b.; characterized in that use is made of an amine having exclusively hydrogen-free carbon atoms in the α-position and a $k_a$, as defined herein, lower than 0,01 $min^{-1}$.

2. A process according to claim 1, characterized in that use is made of t-butylamine.

3. A process according to claim 2, characterized in that the chlorinating liquid substantially contains as solvent N-methyl pyrrolidone.

4. A process according to claim 1, characterized in that the steps a. and c. are carried out at practically the same temperature level.

5. A process according to claim 1, characterized in that it is used for the combined preparation of 1,2-dichloroethane and soda from ethylene and sodium chloride.

**Patentansprüche**

1. Ein Verfahren zur gleichzeitigen Herstellung von chlorierten Kohlenwasserstoffen und einem Carbonat eines Alkalimetalls umfassend die folgenden Verfahrensstufen:

a) Chlorieren eines olefinischen Kohlenwasserstoffs mit einer im wesentlichen nicht-wäßrigen Chlorierflüssigkeit enthaltend Jodung ein Kupfer-und/oder ein Eisenchlorid;

b) Umwandeln eines Alkalimetallchlorids durch Kohlensäuresaturierung in ein Carbonat mit Hilfe eines aliphatischen Amins als alkalische Substanz, wobei als Nebenprodukt das Aminhydrochlorid gebildet wird;

c) Regenerieren der in Verfahrensstufe a) Verwendeten Chlorierflüssigkeit mit dem in Verfahrensstufe b) gebildeten Aminhydrochlorid als Chlorquelle in Gegenwart von Sauerstoff oder einem sauerstoffhaltigen Gas, und Rückführung des wiedergewonnenen Amins in Stufe b), dadurch gekennzeichnet, daß ein Amin mit ausschließlich wasserstoffreien Kohlenstoffatomen in α-Stellung und einem $k_a$-Wert, wie definiert, von weniger als 0,01 $min^{-1}$ verwendet wird.

2. Ein Verfahren gemäß Anspruch 1, dadurch gekennzeichnet, daß t-Butylamin verwendet wird.

3. Ein Verfahren gemäß Anspruch 2, dadurch gekennzeichnet, daß die Chlorierflüssigkeit als Lösungsmittel im wesentlichen N-Methylpyrrolidon enthält.

4. Ein Verfahren gemäß Anspruch 1, dadurch gekennzeichnet, daß die Verfahrensstufen a) und c) in praktisch dem gleichen Temperaturbereich durchgeführt werden.

5. Ein Verfahren gemäß Anspruch 1, dadurch gekennzeichnet, daß es zur gleichzeitigen Herstellung von 1,2-Dichloräthan und Natriumcarbonat aus Äthylen und Natriumchlorid verwendet wird.

**Revendications**

1. Procédé pour la préparation continue d'hydrocarbure chlorés et d'un carbonate d'un métal alcalin, comportant les étapes selon lesquelles

a) on chlore un hydrocarbure oléfinique à l'aide d'un liquide sensiblement non aqueux de chloration contenant de l'iode et du chlorure cuivrique et/ou ferrique;

b) on transforme un chlorure de métal alcalin, par carbonatation, en carbonate à l'aide d'une amine aliphatique comme la matière alcaline, avec formation du chlorhydrate d'amine comme sous-produit;

c) on régénère le liquide de chloration, utilisé dans l'étape a) avec le chlorhydrate d'amine formé à l'étape b) comme source de chlore en présence d'oxygène ou d'un gaz contenant de l'oxygène, et l'on fait revenir l'amine récupérée vers l'étape b); procédé caractérisé en ce qu'on utilise une amine ayant en position α exclusivement des atomes de carbone dépourvus d'hydrogène et présentant une valeur de la constante $k_a$, telle que définie ici, inférieure à 0,01 $min^{-1}$.

2. Procédé selon la revendication 1, caractérisé en ce qu'on utilise la tertiobutylamine.

3. Procédé selon la revendication 2, caractérisé en ce que le liquide de chloration contient essentiellement comme solvant la N-méthylpyrrolidone.

4. Procédé selon la revendication 1, caractérisé en ce qu'on effectue les étapes a) et c) pratiquement au même niveau de température.

5. Procédé selon la revendication 1, caractérisé en ce qu'on l'utilise pour la préparation combinée du 1,2-dichloréthane et de la soude (carbonate de sodium) à partir d'éthylène et de chlorure de sodium.